# EUROPEAN PATENT SPECIFICATION

(11) **EP 1 683 467 B1**
(45) Date of publication and mention of the grant of the patent: **26.01.2011**
(21) Application number: 04792544.1
(22) Date of filing: 18.10.2004
(51) Int. Cl.: A61B 1/05

(54) **DEVICE FOR INTRODUCING INTO EXAMINEE**
VORRICHTUNG ZUR EINFÜHRUNG IN EINE ZU UNTERSUCHENDE PERSON
DISPOSITIF DESTINE A ETRE INTRODUIT DANS LE CORPS D'UN PATIENT EXAMINE

(30) Priority: 24.10.2003 JP 2003364607
(43) Date of publication of application: 26.07.2006
(73) Proprietor: Olympus Corporation, Tokyo 151-0072 (JP)
(72) Inventor: HONDA, Takemitsu, Hino-shi, Tokyo 1910062 (JP)
(74) Representative: von Hellfeld, Axel
(86) International application number: PCT/JP2004/015375
(87) International publication number: WO 2005/039398

(56) References cited:
- JP-A- 2001 251 612
- JP-A- 2003 260 025
- JP-A- 2004 148 124
- US-A1- 2001 051 766
- US-B1- 6 494 827

## Description

### TECHNICAL FIELD

The present invention relates to a body-insertable apparatus which supplies electric power to each electric portion of, for example, a capsule endoscope of a swallow type. More specifically, the present invention relates to a body-insertable apparatus which exhausts electric power of batteries in the apparatus.

### BACKGROUND ART

In recent years, a capsule endoscope equipped with an imaging function and a radio function has appeared in the endoscope field. The capsule endoscope is moved in internal organs such as a stomach and a small intestine (or in body cavities) with peristaltic motion thereof to sequentially perform imaging in the body cavities using the imaging function in an observation period during which the capsule endoscope is swallowed into a subject as a tested body for observation (examination) and is naturally discharged from the living body as the subject.

Image data imaged in the body cavities by the capsule endoscope in the observation period of movement in these internal organs is sequentially transmitted to an external device provided outside the subject by the radio function such as radio communication and is then stored in a memory provided in the external device. Electric power is supplied to drive each electric portion for ensuring the imaging function and the radio function. The driving will be hereinafter called driving of the capsule endoscope. The subject carries the external device having the radio function and the memory function. The subject can be freely moved in the observation period during which the capsule endoscope is swallowed and discharged. After observation, a doctor or a nurse can display the images in the body cavities on a display device such as a display based on the image data stored in the memory of the external device to perform diagnosis.

As such a capsule endoscope, there is one of a swallow type as shown in Patent Document 1. There has been proposed a capsule endoscope having in its inside a reed switch turned on and off by an external magnetic field to control driving of the capsule endoscope and housed in a package including a permanent magnet supplying the external magnetic field. The reed switch provided in the capsule endoscope maintains the off state in an environment in which a magnetic field above a fixed strength is given and is turned on by the lowered strength of the external magnetic field. The capsule endoscope housed in the package is not driven. At swallow, the capsule endoscope is taken out from the package to be away from the permanent magnet. The capsule endoscope is not affected by a magnetic force. The reed switch is in the on state to start driving the capsule endoscope. In such construction, driving of the capsule endoscope housed in the package can be prevented. The capsule endoscope taken out from the package performs imaging by the illumination function and the imaging function and transmits an image signal by the radio function.
Patent Document 1: International Publication Pamphlet WO01/35813

US 6,494,827 B1 discloses an endoscope comprising a battery-powered light source. The battery-powered light source includes a lamp, a battery, a voltage detection unit, a drive circuit and a liquid crystal panel. The voltage detection unit detects a voltage given by the battery, the drive circuit produces a driving signal according to a voltage value signal output by the voltage detection unit and the liquid crystal panel notifies a user of the amount of electrical energy contained in the battery in response to the driving signal output from the drive circuit. Instead of the liquid crystal panel a light-emitted diode may be used to report an amount of electrical energy contained in the battery. In particular, the light emitting diode may be lit, when the battery voltage falls below a predefined value.

### DISCLOSURE OF INVENTION

### PROBLEM TO BE SOLVED BY THE INVENTION

However, the capsule endoscope drives a function execution unit for executing predetermined functions such as the illumination function, the imaging function, and the radio function by supplying electric power from batteries such as button batteries. Use of the batteries for a long time lowers the supplied electric power of the batteries so that the capsule endoscope is at an intermediate potential. The intermediate potential causes a phenomenon such as latchup on the load side of the function execution means so that the capsule endoscope can be in failure mode. A circuit construction disconnecting the batteries and the function execution means in the state of the intermediate potential can be considered. In such construction, the operation of a switching circuit for the disconnection can be unstable to cause the latchup phenomenon. This results in malfunction in the circuits.

The present invention has been achieved in order to solve the above problems. It is an object of this invention to provide a capsule endoscope which exhausts electric power stored in batteries of the body-insertable apparatus to prevent malfunction of the circuits in an intermediate potential state.

### MEANS FOR SOLVING PROBLEM

To solve the problems and achieve the object, a capsule endoscope according to the present invention includes a function execution unit which executes a predetermined function in a subject into which the body-insertable apparatus is introduced; an electric power storage unit which stores driving electric power for driving the function execution unit; a detection unit which detects electric power supplied from the electric power storage unit; and an exhaustion unit which is provided to be separated from the function execution unit and exhausts the electric power of the electric power storage unit based on a detection result of the detection unit.

The capsule endoscope of the invention according to claim 2 further includes a stop unit which stops supply of driving electric power from the electric power storage unit to the function execution unit based on the detection result of the detection unit.

In the capsule endoscope of the invention according to claim 3, the electric power storage unit has a plurality of batteries which are connected in series, and the exhaustion unit exhausts electric power from the respective batteries.

In the capsule endoscope of the invention according to claim 4, the exhaustion unit has resistors and switch devices connectable in parallel with the batteries, respectively, and connects the batteries in parallel with the resistors to exhaust electric power of the batteries by setting the switch devices to the on state based on the detection result of the detection unit.

In the capsule endoscope of the invention according to claim 5, the exhaustion unit has a resistor and a switch device which are connectable in series with the batteries, and connects the batteries in series with the resistor to short-circuit the batteries for exhausting electric power of the batteries by setting the switch device to the on state based on the detection result of the detection unit.

In the capsule endoscope of the invention according to claim 6, the exhaustion unit includes a heat generation unit which generates heat above a predetermined temperature based on the detection result of the detection unit; a resistor connectable in series with the batteries; and a shape-memory member connecting the batteries in series with the resistor based on a critical temperature equal to the predetermined temperature, the batteries being short-circuited to exhaust electric power of the batteries.

In the capsule endoscope of the invention according to claim 7, the function execution unit includes at least an illumination unit which illuminates the inside of the subject; an obtaining unit which obtains information on the inside of the illuminated subject; and a radio transmission unit which radio-transmits the information on the inside of the subject obtained by the obtaining unit to the outside.

### EFFECT OF THE INVENTION

A capsule endoscope according to the present invention stops supply of driving electric power from the electric power storage unit to the function execution unit by the stop unit based on the detection result of electric power supplied from the electric power storage unit, and exhausts the electric power stored in batteries in the body-insertable apparatus by exhaustion unit to prevent malfunction of the circuits in an intermediate potential state.

### BRIEF DESCRIPTION OF DRAWINGS

- FIG. 1: is a system concept view showing the concept of a radio type intra-subject information obtaining system according to the present invention;
- FIG. 2: is a block diagram showing the inner construction in a capsule endoscope according to a first embodiment shown in FIG. 1;
- FIG. 3: is a circuit diagram showing the circuit construction of a system control circuit according to the first embodiment shown in FIG. 2;
- FIG. 4: is a block diagram showing the inner construction of a communication device according to the first embodiment shown in FIG. 1; and
- FIG. 5: is a circuit diagram showing an essential portion of the circuit construction of a system control circuit according to a second embodiment shown in FIG. 2.

### EXPLANATIONS OF LETTERS OR NUMERALS

- 1: subject
- 2: capsule endoscope
- 3: communication device
- 4: display device
- 5: portable recording medium
- 20: light emitting diode (LED)
- 21: LED driving circuit
- 22: charge-coupled device (CCD)
- 23: CCD driving circuit
- 24: RF transmitting unit
- 25: transmitting antenna unit
- 26: system control circuit
- 26a: filed-effect transistor (FET)
- 26b: diode
- 26c: NOT circuit
- 26d, 26e: flip-flop
- 26f, 26g, 26h to 29j: resistor
- 26h, 26i, 26m: switch device
- 27: receiving antenna
- 28: control signal detection circuit
- 29: battery
- 29a to 29c: button battery
- 29d: substrate
- 29e to 29g: shape-memory member
- 30: intra-capsule function execution circuit
- 31: transmission and reception jacket
- 32: external device
- 33: RF receiving unit
- 34: image processing unit
- 35: storage unit
- 36: control signal input unit
- 37: RF transmitting unit circuit
- 38: electric power supplying unit
- A1 to An: receiving antenna
- B1 to Bm: transmitting antenna

### BEST MODE(S) FOR CARRYING OUT THE INVENTION

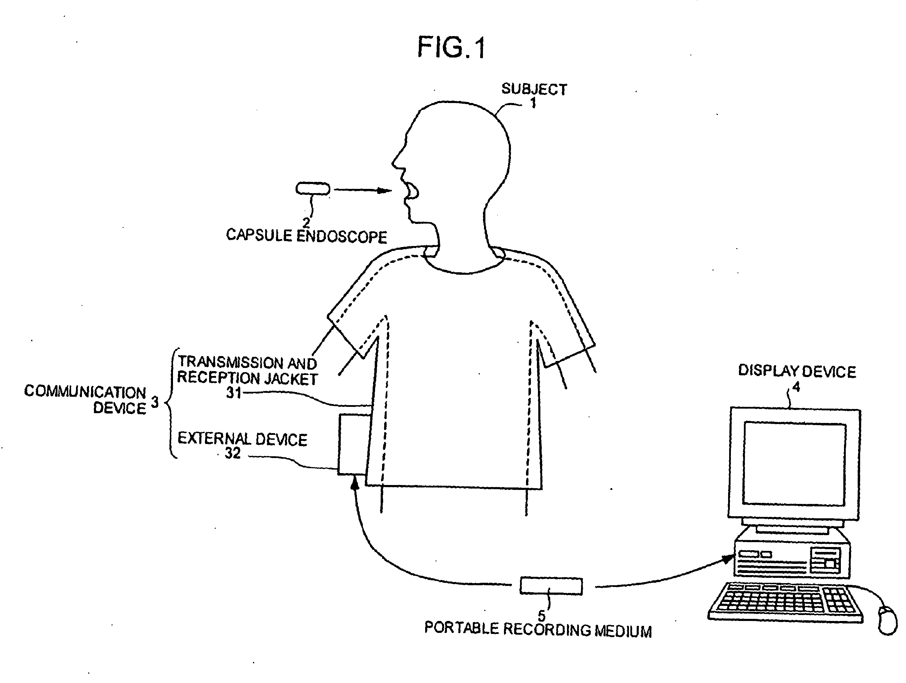

Exemplary embodiments of a body-insertable apparatus according to the present invention will be described in detail below with reference to the drawings of FIGS. 1 to 5. In the following drawings, the same components as those of FIG. 1 are indicated by identical reference numerals for convenience of the description. The present invention is not limited to these embodiments and various modified embodiments can be made in the scope without departing from the subject matter of the present invention.

### First Embodiment

FIG. 1 is a system concept view showing the concept of a wireless in-vivo information obtaining system according to the present invention. In FIG. 1, the wireless in-vivo information obtaining system has a capsule endoscope 2 of a swallow type as a body-insertable apparatus which is introduced into the body cavities of a subject 1, and a communication device 3 as an extracorporeal device arranged outside the subject 1 and radiocommunicating various pieces of information between the communication device 3 and the capsule endoscope 2. The wireless in-vivo information obtaining system also has a display device 4 performing image display based on data received by the communication device 3, and a portable recording medium 5 performing input and output of data between the communication device 3 and the display device 4.

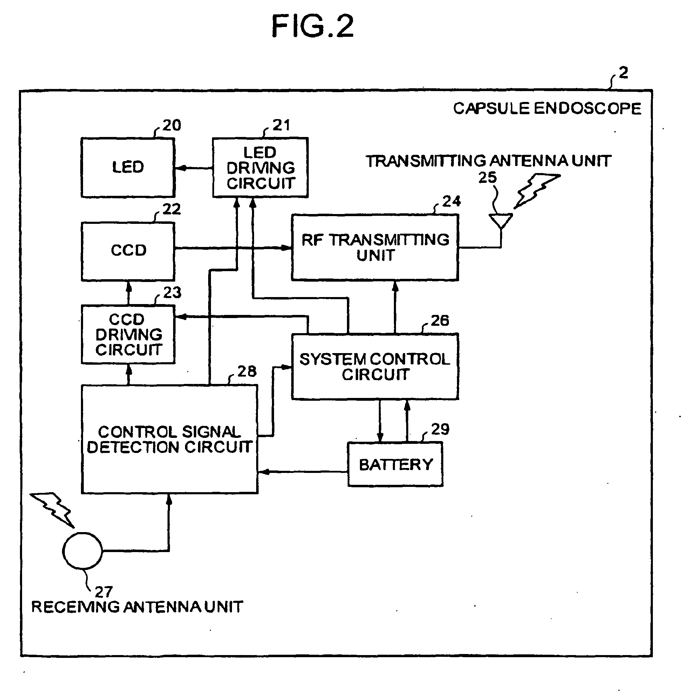

As shown in the block diagram of FIG. 2, the capsule endoscope 2 has a light emitting diode (LED) 20 as an illuminating unit for illuminating an examined portion in the body cavities of the subject 1, an LED driving circuit 21 as first driving means for controlling the driven state of the LED 20, a charge-coupled device (CCD) 22 as obtaining means for imaging an image in the body cavities (in-vivo information) as a reflected light from a region illuminated by the LED 20, a CCD driving circuit 23 as first driving means for controlling the driven state of the CCD 22, an RF transmitting unit 24 modulating the imaged image signal to an RF signal, and a transmitting antenna unit 25 as radio transmitting means for radiotransmitting the RF signal output from the RF transmitting unit 24. The capsule endoscope 2 also has a system control circuit 26 controlling the operation of the LED driving circuit 21, the CCD driving circuit 23, and the RF transmitting unit 24. While the capsule endoscope 2 is introduced into the subject 1, image data of the examined portion illuminated by the LED 20 is obtained by the CCD 22. The obtained image data is converted to an RF signal by the RF transmitting unit 24 and is transmitted to the outside of the subject 1 via the transmitting antenna unit 25.

The capsule endoscope 2 further has a receiving antenna unit 27 as radio receiving means which can receive a radio signal transmitted from the communication device 3; a control signal detection circuit 28 detecting a control signal at a predetermined input level (e.g., reception strength level) from the signal received by the receiving antenna unit 27; and a battery 29 supplying electric power to the system control circuit 26 and the control signal detection circuit 28.

The control signal detection circuit 28 detects the contents of the control signal and outputs the control signal to the LED driving circuit 21, the CCD driving circuit 23, and the system control circuit 26 as needed. The system control circuit 26 has a function of distributing driving electric power supplied from the battery 29 to other components (function execution means).

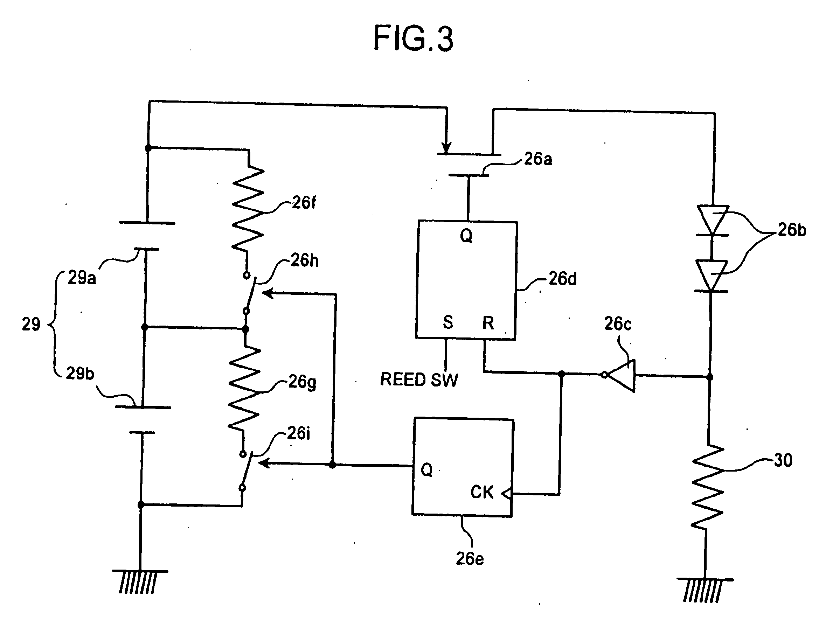

FIG. 3 is a circuit diagram showing the circuit construction of the system control circuit according to a first embodiment shown in FIG. 2. In FIG. 3, the battery 29 is composed of plural (two in the first embodiment) button batteries 29a and 29b.

The system control circuit 26 has an FET (field-effect transistor) 26a whose source terminal is connected to the battery 29, a diode 26b connected to the drain terminal of the FET 26a, a NOT circuit 26c connected to the output terminal of the diode 26b, and a flip-flop 26d reset (R) by an output from the NOT circuit 26c and performing output (Q) to the gate terminal of the FET 26a. The output of the diode 26b is connected to an intra-capsule function execution circuit 30. The flip-flop 26d is set (S) by an input from the above-described reed switch. In the present invention, a switch device can be used in place of a transistor such as an FET. In this embodiment, the imaging function, the illumination function, and the radio function (partially) provided in the capsule endoscope 2 are collectively called a function execution unit for executing predetermined functions. Specifically, the function execution unit for executing predetermined functions except for the system control circuit 26, the receiving antenna unit 27, and the control signal detection circuit 28 is generically called the intra-capsule function execution circuit 30 as needed.

The system control circuit 26 has a flip-flop 26e to which an output of the NOT circuit 26c is input (CK), resistors 26f and 26g connectable in parallel with the button batteries 29a and 29b, respectively, and switch devices 26h and 26i. The switch devices 26h and 26i are in the off state while driving electric power is supplied from the button batteries 29a and 29b to the intra-capsule function execution circuit 30. When no driving electric power is supplied to the intra-capsule function execution circuit 30, the switch devices 26h and 26i are switched to the on state. In such manner, the operation of the switch devices 26h and 26i is controlled by the NOT circuit 26c and the flip-flop 26e. The switch devices 26h and 26i are switched to the on state by the output (Q) from the flip-flop 26e. The button batteries 29a and 29b are connected in parallel with the resistors 26f and 26g, respectively, to exhaust the electric power stored in the button batteries 29a and 29b.

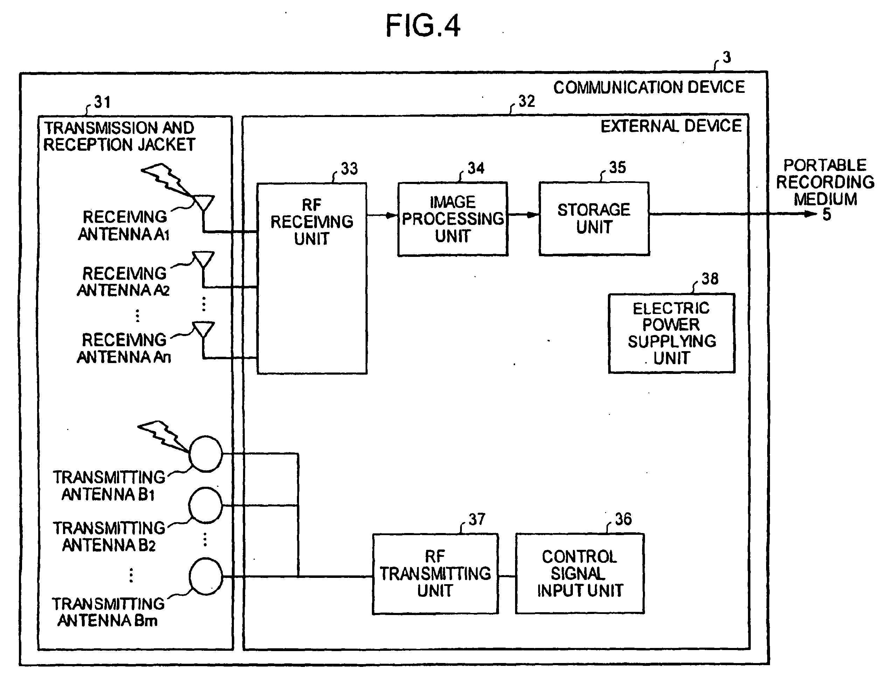

The communication device 3 has a function of the transmission device as the radio transmission means for transmitting a start signal to the capsule endoscope 2, and a function of the reception device as the radio reception means for receiving image data in the body cavities radiotransmitted from the capsule endoscope 2. FIG. 4 is a block diagram showing the inner construction of the communication device 3 according to the first embodiment shown in FIG. 1. In FIG. 4, the communication device 3 has transmission and reception clothes (e.g., transmission and reception jacket) 31 worn by the subject 1 and having plural receiving antennas A1 to An and plural transmitting antennas B1 to Bm, and an external device 32 performing signal processing of a transmitted and received radio signal. It should be noted that n and m indicate any number of antennas set as needed.

The external device 32 has an RF receiving unit 33 performing predetermined signal processing such as demodulation to radio signals received by the receiving antennas A1 to An and extracting image data obtained by the capsule endoscope 2 from the radio signals, an image processing unit 34 performing image processing necessary for the extracted image data, and a storage unit 35 for recording the image-processed image data, and performs signal processing of the radio signals transmitted from the capsule endoscope 2. In this embodiment, the image data is recorded via the storage unit 35 to the portable recording medium 5.

The external device 32 also has a control signal input unit 36 generating a control signal (start signal) for controlling the driven state of the capsule endoscope 2, and an RF transmitting unit circuit 37 converting the generated control signal to a radio frequency to output it. The signal converted by the RF transmitting unit circuit 37 is output to the transmitting antennas B1 to Bm to be transmitted to the capsule endoscope 2. The external device 32 further has an electric power supplying unit 38 having a predetermined capacitor or an AC power source adapter. Each component of the external device 32 uses electric power supplied from the electric power supplying unit 38 as a driving energy.

The display device 4 displays an image in the body cavities imaged by the capsule endoscope 2 and has a configuration such as a workstation performing image display based on data obtained by the portable recording medium 5. Specifically, the display device 4 may directly display an image by a CRT display and a liquid crystal display or may output an image to other medium like a printer.

The portable recording medium 5 can be connected to the external device 32 and the display device 4, and can output or record information when the portable recording medium 5 is inserted into and connected to both. In this embodiment, the portable recording medium 5 is inserted into the external device 32 to record data transmitted from the capsule endoscope 2 while the capsule endoscope 2 is moved in the body cavities of the subject 1. After the capsule endoscope 2 is discharged from the subject 1, that is, after imaging of the inside of the subject 1 is completed, the portable recording medium 5 is taken out from the external device 32 to be inserted into the display device 4. The display device 4 reads the data recorded onto the display device 4. The portable recording medium 5 has a CompactFlash (Registered Trademark) memory and can indirectly perform input and output of data between the external device 32 and the display device 4 via the portable recording medium 5. Unlike the case that the external device 32 and the display device 4 are directly connected by cable, the subject 1 can be freely moved during photographing in the body cavities.

Using the circuit diagram of FIG. 3, the operation of the capsule endoscope 2 will be described. In FIG. 3, the capsule endoscope 2 before being introduced into the subject 1 has in its inside a reed switch, not shown, turned on and off by an external magnetic field and is stored in the state that the capsule endoscope 2 is housed in a package including a permanent magnet supplying the external magnetic field. In this state, the capsule endoscope 2 is not driven.

When the capsule endoscope 2 is taken out from the package at swallow, the capsule endoscope 2 away from the permanent magnet of the package is not affected by a magnet force. The flip-flop 26d is set (S) by an input from the reed switch. The set flip-flop 26d performs the output (Q) to the gate terminal of the FET 26a. The output (Q) flows an electric current between the source and drain terminals of the FET 26a. Electric power from the button batteries 29a and 29b is supplied via the diode 26b to the intra-capsule function execution circuit 30.

A voltage supplied from the button batteries 29a and 29b is "A". Voltages consumed by the FET 26a and the diode are "B" and "C", respectively. A voltage supplied to the intra-capsule function execution circuit 30 is A - (B + C) = X. An intermediate potential Y is set as a threshold value to the NOT circuit 26c. When the voltage X is larger than the intermediate potential Y, that is, (voltage X) > (intermediate potential Y), the switch devices 26h and 26i are in the off state with no output from the NOT circuit 26c.

When the voltage X is equal to or smaller than the intermediate potential Y, that is, (voltage X) ≤ (intermediate potential Y), an output from the NOT circuit 26c resets the flip-flop 26d and the output from the NOT circuit 26c is input to the flip-flop 26e. When the flip-flop 26d is reset, no electric current is flowed between the source and drain terminals. No driving electric power is supplied to the intra-capsule function execution circuit 30. When the output from the NOT circuit 26c is input, the flip-flop 26e performs the output (Q) to switch the switch devices 26h and 26i to the on state. The switch operation connects the button batteries 29a and 29b in parallel with the resistors 26f and 26g, respectively. The resistors 26f and 26g can exhaust the electric power stored in the button batteries 29a and 29b.

In this embodiment, when the voltage supplied from the button batteries is equal to or smaller than the predetermined intermediate potential, supply of the driving electric power to the intra-capsule function execution circuit is stopped. The electric power stored in the button batteries is exhausted by the resistors connected in parallel therewith. A phenomenon such as latchup due to the intermediate potential cannot occur at the load side of the function execution unit. Malfunction of the circuits in the intermediate potential state can be prevented.

### Second Embodiment

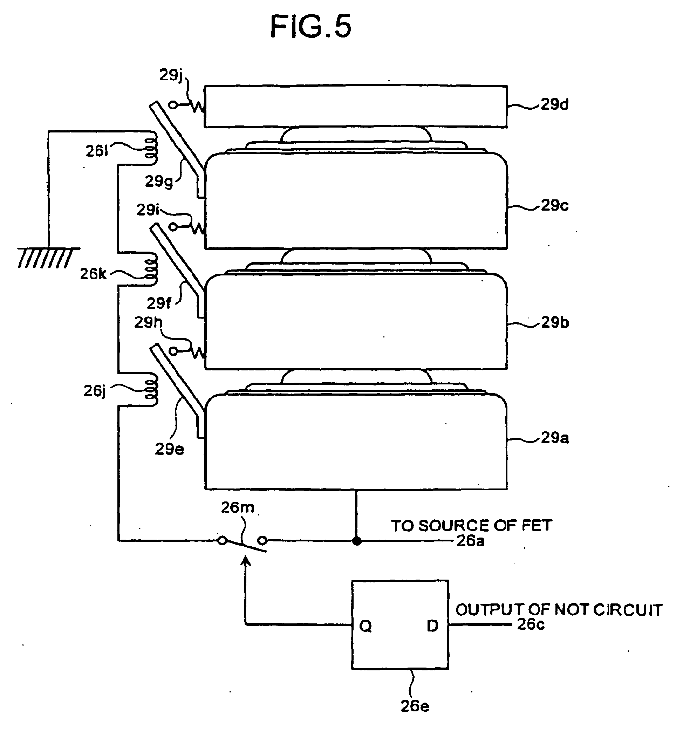

FIG. 5 is a circuit diagram showing an essential portion of the circuit construction of the system control circuit according to a second embodiment shown in FIG. 2. In FIG. 5, the battery 29 of the second embodiment has three button batteries 29a to 29c stacked in series and is grounded to a conductive substrate 29d provided in the capsule endoscope 2.

As in the first embodiment, the system control circuit according to the second embodiment has the FET 26a, the diode 26b, the NOT circuit 26c, the flip-flop 26d, and a flip-flop 26e connected to the button battery 29a. Further, the system control circuit according to the second embodiment has shape-memory members 29e to 29g made of conductive members arranged in positive pole cases of the button batteries 29a to 29c stacked in series, and resistors 29h to 29j arranged on the button batteries 29b and 29c and the substrate 29d.

The button battery 29a has, in its positive pole, heat coils 26j to 261 connectable in series therewith and a switch device 26m. The switch device 26m is in the off state while driving electric power is supplied from the button batteries 29a to 29c to the intra-capsule function execution circuit 30. The switch device 26m is switched to the on state while no driving electric power is supplied to the intra-capsule function execution circuit 30. In such manner, the operation of the switch device 26m is controlled by the NOT circuit 26c and the flip-flop 26e. The switch device 26m is switched to the on state by the output (Q) from the flip-flop 26e to connect the button batteries 29a to 29c in series with the heat coils 26j to 261.

When an electric current flows, the heat coils 26j to 261 generate heat above a predetermined temperature, i.e., at 40 to 45°C slightly higher than the temperature of the subject. The shape-memory members 29e to 29g are made of a shape-memory alloy or a shape-memory resin which uses the predetermined temperature as a critical temperature and is recovered to a memory shape above such a critical temperature. When the shape-memory members 29e to 29g are recovered to the memory shape, they are electrically connected to the resistors 29h to 29j arranged on the adjacent button batteries 29b and 29c and the substrate 29d to short-circuit the button batteries 29a to 29c. In the second embodiment, the resistors of the resistors 29h to 29j are adjusted to prevent an overcurrent from occurring in order to avoid heat generation due to the overcurrent flowed to the button batteries 29a to 29c with the short circuit. The resistors 29h to 29j are made of conductive members whose resistances are adjusted, e.g., of rubber or plastic.

When the voltage X supplied from the intra-capsule function execution circuit 30 is equal to or smaller than the intermediate potential Y set by the NOT circuit 26c, an output from the NOT circuit 26c stops supply of driving electric power to the intra-capsule function execution circuit 30 and the flip-flop 26e performs the output (Q) to switch the switch device 26m to the on state. The switch operation connects the button batteries 29a and 29b in series with the heat coils 26j to 261. The heat coils 26j to 261 are heat generated at a predetermined temperature. By the heat generation, the shape-memory members 29e to 29g are recovered to the memory shape to be electrically connected to the resistors 29h to 29j for short-circuiting the button batteries 29a to 29c.

In this embodiment, when the voltage supplied from the button batteries is equal to or smaller than the predetermined intermediate potential, supply of the driving electric power to the intra-capsule function execution circuit is stopped. The button batteries are short-circuited via the resistors to exhaust the electric power stored in the button batteries. As in the first embodiment, malfunction of the circuits in the intermediate potential state can be prevented.

In the present invention, the switch device and the resistors connectable in series are arranged between the button batteries 29a to 29c and the substrate 29d shown in FIG. 5. When the switch device is switched to the on state by the operation control of the flip-flop 26e, the button batteries 29a to 29c are electrically connected in series with the resistors to short-circuit the button batteries 29a to 29c. In this case, the switch device and the resistors are patterned to an insulating resin film. The resin film is bonded to the positive pole cases of the button batteries 29a to 29c. The contacts of the switch device and the resistors are electrically connected with the positive pole cases.

In this case, as in the second embodiment, when the voltage supplied from the button batteries is equal to or smaller than the predetermined intermediate potential, the button batteries are short-circuited via the resistors to exhaust the electric power stored in the button batteries. Malfunction of the circuits in the intermediate potential state can be prevented. No heat occurs in the capsule endoscope. A few number of components can exhaust the electric power stored in the button batteries.

### INDUSTRIAL APPLICABILITY

As described above, the body-insertable apparatus according to the present invention is useful for a medical observation device introduced into a human body and observing an examined portion, and in particular, is suitable for preventing malfunction of the circuits in the device in the intermediate potential state.

## Claims

1. A capsule endoscope (2) comprising:
a function execution unit (20, 22, 24) which executes a predetermined function in a subject (1) into which the capsule endoscope (2) is introduced;
an electric power storage unit (29) which stores driving electric power for driving the function execution unit (20, 22, 24);
a detection unit (28) which detects electric power supplied from the electric power storage unit (29); **characterized by**
an exhaustion unit (26) which is provided to be separated from the function execution unit (20, 22, 24) and exhausts the electric power of the electric power storage unit (29) based on a detection result of the detection unit (28).

2. The capsule endoscope (2) according to claim 1, further comprising a stop unit (26h, 26i; 29e, 29f, 29g) which stops supply of driving electric power from the electric power storage unit (29) to the function execution unit (20, 22, 24) based on the detection result of the detection unit (28).

3. The capsule endoscope (2) according to claim 1 or 2, wherein
the electric power storage unit (29) has a plurality of batteries (29a, 29b, 29c) which are connected in series, and
the exhaustion unit (26) exhausts electric power from the respective batteries (29a, 29b, 29c).

4. The capsule endoscope (2) according to claim 3, wherein the exhaustion unit (26) has resistors (26f, 26g) and switch devices (26h, 26i) connectable in parallel with the batteries(29a, 29b, 29c), respectively, and connects the batteries (29a, 29b, 29c) in parallel with the resistors(26f, 26g; 29h, 29i, 29j) to exhaust electric power of the batteries (29a, 29b, 29c) by setting the switch devices (26h, 261; 29e, 29f, 29g) to the on state based on the detection result of the detection unit (28).

5. The capsule endoscope (2) according to claim 3, wherein the exhaustion unit (26) has a resistor (29h, 29i, 29j) and a switch device (29e, 29f, 29g) which are connectable in series with the batteries (29a, 29b, 29c), and connects the batteries (29a, 29b, 29c) in series with the resistor (29h, 29i, 29j) to short-circuitbatteries (29a, 29b, 29c) for exhausting electric power of the batteries (29a, 29b, 29c) by setting the switch device to the on state based on the detection result of the detection unit (28).

6. The capsule endoscope (2) according to claim 3, wherein the exhaustion unit (26) includes
a heat generation unit (26j, 26k, 261) which generates heat above a
a resistor (29h, 29i, 29j) connectable in series with the batteries (29a, 29b, 29c); and
a shape-memory member (29e, 29f, 29g) connecting the batteries in series with the resistor (29h, 291, 29j) based on a critical temperature equal to the predetermined temperature,
the batteries (29a, 29b, 29c) being short-circuited to exhaust electric power of the batteries (29a, 29b, 29c).

7. The capsule endoscope (2) according to claim 1 or 2, wherein the function execution unit (20, 22, 24) includes at least
an illumination unit (20) which illuminates the inside of the subject (1);
an obtaining unit (22) which obtains information on the inside of the illuminated subject; and
a radio transmission unit (24) which radio-transmits the information on the inside of the subject (1) obtained by the obtaining unit (22) to the outside.

## Patentansprüche

1. Kapselendoskop (2), aufweisend:
eine Funktionsdurchführungseinheit (20, 22, 24), welche eine vorbestimmte Funktion in einem Objekt (1) durchführt, in welches das Kapselendoskop (2) eingebracht ist;
eine Speichereinheit (29) für elektrische Energie, welche elektrische Antriebsenergie zum Betreiben der Funktionsdurchführungseinheit (20, 22, 24) speichert;
eine Erkennungseinheit (28), welche die elektrische Energie erkennt, die von der Speichereinheit (29) für elektrische Energie geliefert wird, **gekennzeichnet durch**
eine Verbrauchseinheit (26), welche dazu eingerichtet ist, von der Funktionsdurchführungseinheit (20, 22, 24) getrennt zu werden und die elektrische Energie der Speichereinheit (29) für elektrische Energie basierend auf einem Erkennungsergebnis der Erkennungseinheit (28) aufbrauch

2. Kapselendoskop (2) nach Anspruch 1, weiterhin aufweisend eine Stoppeinheit (26h, 26i; 29e, 29f, 29g), welche die Zufuhr von elektrischer Antriebsenergie von der Speichereinheit (29) für elektrische Energie an die Funktionsdurchführungseinheit (20, 22, 24) auf der Grundlage des Erkennungsergebnisses von der Erkennungseinheit (28) stoppt.

3. Kapselendoskop nach Anspruch 1 oder 2, wobei
die Speichereinheit (29) für elektrische Energie eine Mehrzahl von Batterien (29a, 29b, 29c) aufweist, die in Serie verbunden sind, und
die Verbrauchseinheit (26) elektrische Energie aus den jeweiligen Batterien (29a, 29b, 29c) aufbraucht.

4. Kapselendoskop (2) nach Anspruch 3, wobei die Verbrauchseinheit (26) Widerstände (26f, 26g) bzw. Schaltvorrichtungen (26h, 26i) aufweist, die parallel zu den Batterien (29a, 29b, 29c), verbindbar sind und die Batterien (29a, 29b, 29c) parallel zu den Widerständen (26f, 26g; 29h, 29i, 29j) schaltet, um elektrische Energie der Batterien (29a, 29b, 29c) aufzubrauchen, indem die Schaltvorrichtungen (26h, 26i; 29e, 29f, 29g) basierend auf dem Erkennungsergebnis der Erkennungseinheit (28) in den Ein-Zustand geschaltet werden.

5. Kapselendoskop (2) nach Anspruch 3, wobei die Verbrauchseinheit (26) einen Widerstand (29h, 29i, 29j) und eine Schaltvorrichtung (29e, 29f, 29g) aufweist, die seriell mit den Batterien (29a, 29b, 29c) verbindbar ist und die Batterien (29a, 29b, 29c) in Serie mit dem Widerstand (26f, 26g; 29h, 29i, 29j) schaltet, um die Batterien (29a, 29b, 29c) kurzzuschließen, um die elektrische Energie der Batterien (29a, 29b, 29c) aufzubrauchen, indem die Schaltvorrichtung basierend auf dem Erkennungsergebnis der Erkennungseinheit (28) in den Ein-Zustand geschaltet wird.

6. Kapselendoskop (2) nach Anspruch 3, wobei die Verbrauchseinheit (26) aufweist:
eine Wärmeerzeugungseinheit (26j, 26k, 261), welche basierend auf dem Erkennungsergebnis von der Erkennungseinheit (28) Wärme über einer vorbestimmten Temperatur erzeugt;
einen Widerstand (29h, 29i, 29j), der in Serie mit den Batterien (29a, 29b, 29c) verbindbar ist; und
ein Form-Gedächtnisteil (29e, 29f, 29g), welches basierend auf einer einer vorbestimmten Temperatur entsprechenden kritischen Temperatur die Batterien in Serie mit dem Widerstand (29h, 29i, 29j) verbindet,
so dass die Batterien (29a, 29b, 29c) kurzgeschlossen werden, um die elektrische Energie der Batterien (29a, 29b, 29c) aufzubrauchen.

7. Kapselendoskop (2) nach Anspruch 1 oder 2, wobei die Funktionsdurchführungseinheit (20, 22, 24) zumindest aufweist:
eine Beleuchtungseinheit (20), die das Innere des Objekts (1) ausleuchtet;
eine Erlangungseinheit (22), welche Informationen aus dem Inneren des ausgeleuchteten Objekts erlangt; und
eine Funkübertragungseinheit (24) welche die von der Erlangungseinheit (22) erlangte Information über das Innere des Objekts (1) über Funk nach außen überträgt.

## Revendications

1. Endoscope à capsule (2) comprenant:
une unité d'exécution de fonction (20, 22, 24) qui exécute une fonction prédéterminée dans un sujet (1) dans lequel endoscope à capsule (2) est introduit;
une unité de stockage d'énergie électrique (29) qui stocke une énergie électrique d'entraînement pour entraîner l'unité d'exécution de fonction (20, 22, 24);
une unité de détection (28) qui détecte une énergie électrique délivrée par l'unité de stockage d'énergie électrique (29); et **caractérisé par**
une unité d'épuisement (26) qui est prévue de façon à être séparée de l'unité d'exécution de fonction (20, 22, 24) et épuise l'énergie électrique de l'unité de stockage d'énergie électrique (29) sur la base d'un résultat de détection de l'unité de détection (28).

2. Endoscope à capsule (2) selon la revendication 1, comprenant en outre une unité d'arrêt (26h, 26i; 29e, 29f, 29g) qui arrête la délivrance de l'énergie électrique d'entraînement de l'unité de stockage d'énergie électrique (29) à l'unité d'exécution de fonction (20, 22, 24) sur la base du résultat de détection de l'unité de détection (28).

3. Endoscope à capsule (2) selon la revendication 1 ou 2, dans lequel
l'unité de stockage d'énergie électrique (29) a une pluralité de batteries (29a, 29b, 29c) qui sont connectées en série, et
l'unité d'épuisement (26) épuise l'énergie électrique des batteries (29a, 29b, 29c) respectives.

4. Endoscope à capsule (2) selon la revendication 3, dans lequel l'unité d'épuisement (26) a des résistances (26f, 26g) et des dispositifs de commutation (26h, 26i) pouvant être connectés en parallèle avec les batteries (29a, 29b, 29c), respectivement, et connecte les batteries (29a, 29b, 29c) en parallèle avec les résistances (26f, 26g ; 29h, 29i, 29j) pour épuiser l'énergie électrique des batteries (29a, 29b, 29c) en mettant les dispositifs de commutation (26h, 26i ; 29e, 29f, 29g) en l'état activé sur la base du résultat de détection de l'unité de détection (28).

5. Endoscope à capsule (2) selon la revendication 3, dans lequel l'unité d'épuisement (26) a une résistance (29h, 29i, 29j) et un dispositif de commutation (29e, 29f, 29g) qui peuvent être connectés en série avec les batteries (29a, 29b, 29c), et connecte les batteries (29a, 29b, 29c) en série avec la résistance (29h, 29i, 29j) pour court-circuiter les batteries (29a, 29b, 29c) pour épuiser l'énergie électrique des batteries (29a, 29b, 29c) en mettant le dispositif de commutation en l'état activé sur la base du résultat de détection de l'unité de détection (28).

6. Endoscope à capsule (2) selon la revendication 3, dans lequel l'unité d'épuisement (26) inclut
une unité de génération de chaleur (26j, 26k, 26l) qui génère une chaleur supérieure à une température prédéterminée sur la base du résultat de détection de l'unité de détection (28);
une résistance (29h, 29i, 29j) pouvant être connectée en série avec les batteries (29a, 29b, 29c); et
un élément à mémoire de forme (29e, 29f, 29g) connectant les batteries en série avec la résistance (29h; 29i; 29j) sur la base d'une température critique égale à la température prédéterminée,
les batteries (29a, 29b, 29c) étant court-circuitées pour épuiser l'énergie électrique des batteries (29a, 29b, 29c).

7. Endoscope à capsule (2) selon la revendication 1 ou 2, dans lequel l'unité d'exécution de fonction (20, 22, 24) inclut au moins
une unité d'éclairage (20) qui éclaire l'intérieur du sujet (1);
une unité d'obtention (22) qui obtient des informations sur l'intérieur du sujet éclairé; et
une unité de transmission radio (24) qui transmet par radio les informations sur l'intérieur du sujet (1) obtenues par l'unité d'obtention (22) jusqu'à l'extérieur.
